(19) Europäisches Patentamt
European Patent Office
Office européen des brevets

(11) Veröffentlichungsnummer: **0 009 637**
**A1**

(12) # EUROPÄISCHE PATENTANMELDUNG

(21) Anmeldenummer: **79103252.7**

(22) Anmeldetag: **03.09.79**

(51) Int. Cl.³: **C 07 C 121/75**
C 07 D 317/60, A 01 N 37/34

(30) Priorität: **04.09.78 CH 9292/78**
**03.04.79 CH 3089/79**

(43) Veröffentlichungstag der Anmeldung:
**16.04.80 Patentblatt 80/8**

(84) Benannte Vertragsstaaten:
**AT BE CH DE FR GB IT NL**

(71) Anmelder: **CIBA-GEIGY AG**
**Patentabteilung Postfach**
**CH-4002 Basel(CH)**

(72) Erfinder: **Drabek, Jozef, Dr.**
**Benkenstrasse 12**
**CH-4104 Oberwil(CH)**

(72) Erfinder: **Ackermann, Peter, Dr.**
**Reichensteinerstrasse 12**
**CH-4153 Reinach(CH)**

(72) Erfinder: **Farooq, Saleem, Dr.**
**Im Schaiengarten 2**
**CH-4107 Ettingen(CH)**

(72) Erfinder: **Gsell, Laurenz, Dr.**
**Maiengasse 56**
**CH-4056 Basel(CH)**

(72) Erfinder: **Kristiansen, Odd, Dr.**
**Delligrabenstrasse 7**
**CH-4313 Möhlin(CH)**

(72) Erfinder: **Wehrli, Rudolf, Dr.**
**Dianastrasse 2**
**CH-4310 Rheinfelden(CH)**

(74) Vertreter: **Zumstein sen., Fritz, Dr. et al,**
**Bräuhausstrasse 4**
**D-8000 München 2(DE)**

(54) **Alpha-phenyl-alpha-isopropylacetate, Verfahren zu ihrer Herstellung, sie enthaltende Schädlingsbekämpfungsmittel und die Verwendung dieser Acetate in der Schädlingsbekämpfung.**

(57) Die neuen α-Phenyl-α- isopropylacetate der Formel

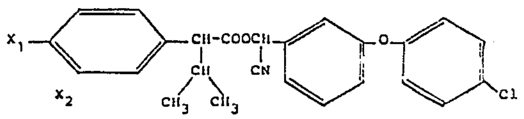

(I)

worin $X_1$ Wasserstoff, Halogen, $C_1$-$C_4$-Alkyl oder $C_1$-$C_4$-Alkoxy, $X_2$ Wasserstoff oder Halogen oder $X_1$ und $X_2$ zusammen Methylendioxy bedeuten, wobei $X_1$ und $X_2$ insbesondere je für Wasserstoff oder Chlor stehen, eignen sich zur Bekämpfung tierischer oder pflanzlicher Schädlinge, insbesondere von Insekten und Vertretern der Ordnung Akarina. Sie lassen sich z.B. durch Umsetzung einer Verbindung der Formel

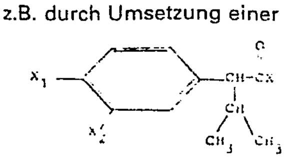

(IV)

worin $X_1$ und $X_2$ die oben angegebene Bedeutung haben und X für ein Halogenatom steht, in Gegenwart eines säurebindenden Mittels mit der Verbindung der Formel

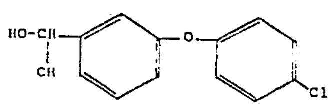

(V)

herstellen.

BAD ORIGINAL

EP 0 009 637 A1

Croydon Printing Company Ltd

BEZEICHNUNG GEÄNDERT
siehe Titelseite

5-12013/1+2/+

## Neue Ester

Die vorliegende Erfindung betrifft α-Phenyl-α-isopropyl-acetate, Verfahren zu ihrer Herstellung und ihre Verwendung in der Schädlingsbekämpfung.

Die α-Phenyl-α-isopropyl-acetate haben die Formel

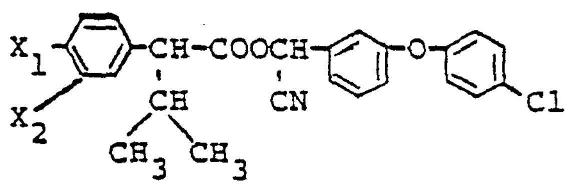

(I)

worin $X_1$ Wasserstoff, Halogen, $C_1$-$C_4$-Alkyl oder $C_1$-$C_4$-Alkoxy,

$X_2$ Wasserstoff oder Halogen oder

$X_1$ und $X_2$ zusammen Methylendioxy bedeuten.

Unter Halogen ist dabei Fluor, Chlor, Brom oder Jod, insbesondere aber Chlor, zu verstehen.

Bei $X_1$ kommen beispielsweise als Alkyl- oder Alkoxygruppen in Frage: Methyl, Methoxy, Aethyl, Aethoxy, Propyl, Propoxy, Isopropyl, Isopropoxy, n-, i-, sek.-, tert.-Butyl. Wegen ihrer Wirkung

bevorzugt sind Verbindungen der Formel I, worin $X_1$ und $X_2$ je Wasserstoff oder Chlor bedeuten.

Die Verbindungen der Formel I können nach an sich bekannten Methoden, z.B. wie folgt hergestellt werden.

1) 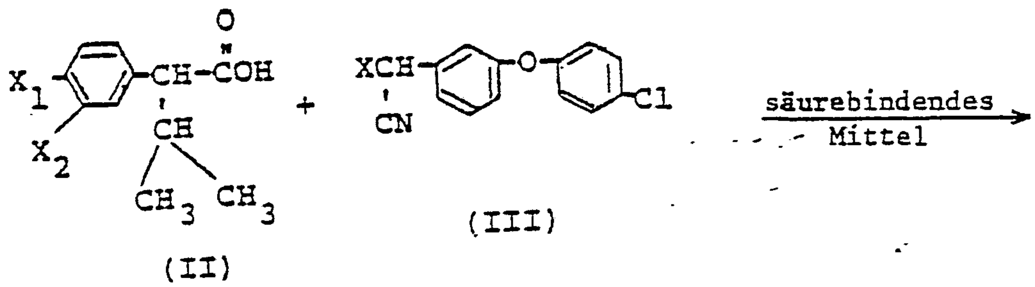

I

(II) (III)

2) 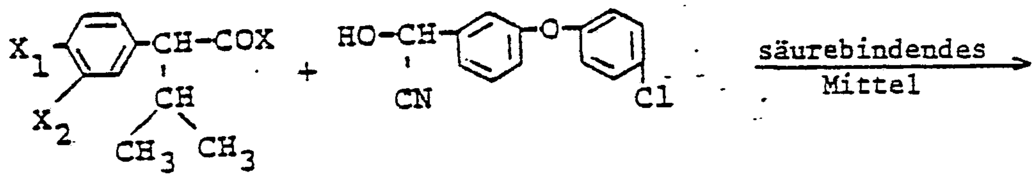

I

(IV) (V)

3) 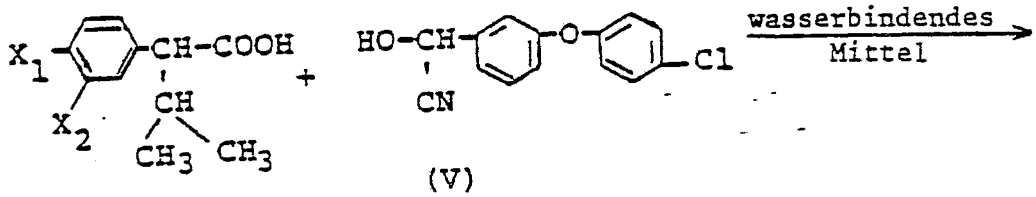

I

(II) (V)

4) 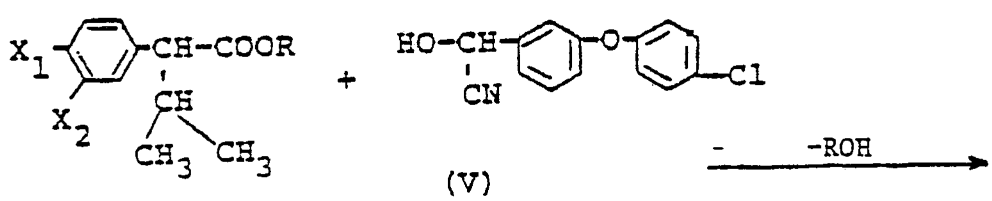

I

(VI) (V)

In den Formeln II bis VI haben $X_1$ und $X_2$, die für die Formel I angegebene Bedeutung.

In den Formeln III und IV steht X für ein Halogenatom, insbesondere Chlor oder Brom, und in der Formel VI steht R für $C_1$-$C_4$-Alkyl, insbesondere für Methyl oder Aethyl. Als säurebindendes Mittel für die Verfahren 1 und 2 kommen insbesondere tertiäre Amine, wie Trialkylamin und Pyridin, ferner Hydroxide, Oxide, Carbonate und Bicarbonate von Alkali- und Erdalkalimetallen sowie Alkalimetallalkoholate wie z.B. Kalium-t.butylat und Natriummethylat in Betracht. Als wasserbindendes Mittel für das Verfahren 3 kann z.B. Dicyclohexylcarbodiimid verwendet werden. Die Verfahren 1 bis 4 werden bei einer Reaktionstemperatur zwischen -10 und 120°C, meist zwischen 20 und 80°C bei normalem oder erhöhtem Druck und vorzugsweise in einem inerten Lösungs- oder Verdünnungsmittel durchgeführt. Als Lösungs- oder Verdünnungsmittel eignen sich z.B. Aether und ätherartige Verbindungen wie Diäthyläther, Dipropyläther, Dioxan, Dimethoxyäthan und Tetrahydrofuran; Amide wie N,N-dialkylierte Carbonsäureamide; aliphatische, aromatische sowie halogenierte Kohlenwasserstoffe, insbesondere Benzol, Toluol, Xylole, Chloroform und Chlorbenzol; Nitrile wie Acetonitril; Dimethylsulfoxid und Ketone wie Aceton und Methyläthylketon.

Die Ausgangsstoffe der Formeln II bis VII sind bekannt oder können analog bekannten Methoden hergestellt werden.

Die Verbindungen der Formel I liegen als Gemisch von verschiedenen optisch aktiven Isomeren vor, wenn bei der Herstellung nicht einheitlich optisch aktive Ausgangsmaterialien verwendet wurden. Die verschiedenen Isomerengemische können nach bekannten Methoden in die einzelnen Isomeren aufgetrennt werden. Unter der Verbindung der Formel I versteht man sowohl die einzelnen Isomeren, als auch deren Gemische.

Die Verbindungen der Formel I eignen sich zur Bekämpfung von verschiedenartigen tierischen und pflanzlichen Schädlingen.

Insbesondere eignen sich die Verbindungen der Formel I zur Bekämpfung von Insekten, phytopathogenen Milben und von Zecken z.B. der Ordnungen Lepidoptera, Coleoptera, Homoptera, Heteroptera, Diptera, Acarina, Thysanoptera, Orthoptera, Anoplura, Siphonaptera, Mallophaga, Thysanura, Isoptera, Psocoptera und Hymenoptera.

Vor allem eignen sich Verbindungen der Formel I zur Bekämpfung von pflanzenschädigenden Insekten, insbesondere pflanzenschädigenden Frassinsekten, in Zier- und Nutzpflanzen, insbesondere in Baumwollkulturen (z.B. gegen Spodoptera littoralis und Heliothis virescens) und Gemüsekulturen (z.B. gegen Leptinotarsa decemlineata und Myzus persicae.

Wirkstoffe der Formel I zeignen auch eine sehr günstige Wirkung gegen Fliegen wie z.B. Musca domestica und Mückenlarven.

Die Verbindungen der Formel (I) weisen insbesondere auch eine ausgezeichnete Wirkung gegen keratinfressende Insekten auf, wie z.B. gegen keratinfressende Larven von Leptidoptera, z.B. Tineola spec. und Tinea spec. sowie gegen keratinfressende Larven von Coleoptera, z.B. Anthrenus spec. und Attagenus spec. auf. Die Wirkstoffe der Formel (I) eignen sich vorzüglich zum Schützen von keratinischen bzw. keratinhaltigem Material gegen Insektenfrass, insbesondere zur wasch- und lichtechten Ausrüstung gegen Insekten, insbesondere zur Motten- und Käferechtausrüstung von derartigen Materialien. Es kann keratinisches bzw. keratinhaltiges Material sowohl in rohem als auch in verarbeitetem Zustand ausgerüstet werden, z.B. rohe oder verarbeitete Schafwolle, Produkte aus anderen Tierhaaren, Felle, Pelze und Federn.

Praktisch besonders wichtig ist die Wirksamkeit der im erfindungsgemässen Verfahren eingesetzten Verbindungen der Formel (I) gegen die Larven der Kleidermotte (Tineola bisselliela) und Pelzmotte (Tinea pellionella) sowie auch gegen die Larven der Pelz- und Teppichkäfer (Attagenus spec. bzw. Anthrenus spec.) Bevorzugt wird das er-

findungsgemässe Verfahren daher einerseits zum Schützen von Textilien aus Wolle, z.B. von Wolldecken, Wollteppichen, Wollwäsche, Wollkleidern und Wirkwaren bzw. von wollhaltigen Textilien, wie Mischgeweben, deren eine Komponente Wolle ist, z.B. Mischgewebe aus Wolle und andere Naturfasern, vorzugsweise Baumwolle oder aus Wolle und Kunstfasern, andererseits auch zum Schützen von Pelzen und Fellen vor dem Befall durch die erwähnten Schädlinge eingesetzt.

Die akarizide bzw. insektizide Wirkung lässt sich durch Zusatz von anderen Insektiziden und/oder Akariziden wesentlich verbreitern und an gegebene Umstände anpassen. Als Zusätze eignen sich z.B. org. Phosphorverbindungen; Nitrophenole und deren Derivate; Formamidine; Harnstoffe; andere pyrethrinartige Verbindungen sowie Karbamate und chlorierte Kohlenwasserstoffe.

Mit besonderem Vorteil werden Verbindungen der Formel I auch mit Substanzen kombiniert, welche einen synergistischen oder verstärkenden Effekt auf Pyrethroide ausüben. Beispiele solcher Verbindungen sind u.a. Piperonylbutoxid, Propinyläther, Propinyloxim, Propinylcarbamate und Propinylphosphonate, 2-(3,4-Methylendioxyphenoxy)-3,6,9-trioxaundecan (Sesamex resp. Sesoxane), S,S,S-Tributylphosphorotrithioate, 1,2-Methylendioxy-4-(2-(octylsulfinyl)-propyl)-benzol.

Verbindungen der Formel I können für sich allein oder zusammen mit geeigneten Trägern und/oder Zuschlagstoffen eingesetzt werden. Geeigneten Zuschlagstoffe können fest oder flüssig sein und entsprechen den in der Formulierungstechnik üblichen Stoffen wie z.B. natürlichen oder regenerierten Stoffen, Lösungs-, Dispergier-, Netz-, Haft-, Verdickungs-, Binde- und/oder Düngemitteln.

Die Herstellung erfindungsgemässer Mittel erfolgt in an sich bekannter Weise durch inniges Vermischen und/oder Vermahlen der Wirkstoffe der Formel I mit den geeigneten Trägerstoffe, gegebenenfalls unter Zusatz von gegenüber den Wirkstoffen inerten Dispergier- oder Lösungsmitteln. Die Wirkstoffe können in den folgenden Aufarbeitungsformen vorliegen und angewendet werden:

Feste Aufarbeitungsformen: Stäubemittel, Streumittel, Granulate (Umhüllungsgranulate, Imprägnierungsgranulate und Homogengranulate);

Flüssige Aufarbeitungsformen:

a) in Wasser dispergierbare Wirkstoffkonzentrate: Spritzpulver (wettable powders), Pasten, Emulsionen;

b) Lösungen.

Der Gehalt an Wirkstoff in den oben beschriebenen Mitteln liegt zwischen 0,1 bis 95%, dabei ist zu erwähnen, dass bei der Applikation aus dem Flugzeug oder mittels anderer geeigneter Applikationsgeräte Konzentrationen bis zu 99,5% oder sogar reiner Wirkstoff eingesetzt werden können. Die Wirkstoffe der Formel I können beispielsweise wie folgt formuliert werden (Teile bedeuten Gewichtsteile):

Stäubemittel: Zur Herstellung eines a) 5%igen und b) 2%igen Stäubemittels werden die folgenden Stoffe verwendet:

a) 5 Teile Wirkstoff,
95 Teile Talkum;

b) 2 Teile Wirkstoff,
1 Teil hochdisperse Kieselsäure,
97 Teile Talkum.

Der Wirkstoff wird mit den Trägerstoffen vermischt und vermahlen.

Granulat: Zur Herstellung eines 5%igen Granulates werden die folgenden Stoffe verwendet:

| | | |
|---|---|---|
| 5 | Teile | Wirkstoff, |
| 0,25 | Teile | Epichlorhydrin |
| 0,25 | Teile | Cetylpolyglykoläther, |
| 3,50 | Teile | Polyäthylenglykol, |
| 91 | Teile | Kaolin (Korngrösse 0,3-0,8 mm). |

Die Aktivsubstanz wird mit Epichlorhydrin vermischt und mit 6 Teilen Aceton gelöst, hierauf wird Polyäthylenglykol und Cetylpolyglykoläther zugesetzt. Die so erhaltene Lösung wird auf Kaolin aufgesprüht und anschliessend das Aceton im Vakuum verdampft.

Spritzpulver: Zur Herstellung eines a) 40%igen, b) und c) 25%igen, d) 10%igen Spritzpulvers werden folgende Bestandteile verwendet:

| | | | |
|---|---|---|---|
| a) | 40 | Teile | Wirkstoff, |
| | 5 | Teile | Ligninsulfonsäure-Natriumsalz, |
| | 1 | Teil | Dibutylnaphthalinsulfonsäure-Natriumsalz, |
| | 54 | Teile | Kieselsäure; |
| b) | 25 | Teile | Wirkstoff, |
| | 4,5 | Teile | Calcium-Ligninsulfonat, |
| | 1,9 | Teile | Champagne-Kreide/Hydroxyäthylcellulose-Gemisch (1:1), |
| | 1,5 | Teile | Natrium-dibutyl-naphthalinsulfonat, |
| | 19,5 | Teile | Kieselsäure, |
| | 19,5 | Teile | Champagne-Kreide, |
| | 28,1 | Teile | Kaolin; |

c) 25 Teile Wirkstoff,
2,5 Teile Isooctylphenoxy-polyäthylen-äthanol,
1,7 Teile Champagne-Kreide/Hydroxyäthylcellulose-Gemisch (1:1),
8,3 Teile Natriumaluminiumsilikat,
16,5 Teile Kieselgur,
46 Teile Kaolin;

d) 10 Teile Wirkstoff,
3 Teile Gemisch der Natriumsalze von gesättigten Fettalkoholsulfaten,
5 Teile Naphthalinsulfonsäure/Formaldehyd-Kondensat,
82 Teile Kaolin.

Der Wirkstoff wird in geeigneten Mischern mit dem Zuschlagstoff innig vermischt und auf entsprechenden Mühlen und Walzen vermahlen. Man erhält Spritzpulver, die sich mit Wasser zu Suspensionen jeder gewünschten Konzentration verdünnen lassen.

Emulgierbare Konzentrate: Zur Herstellung eines a) 10%igen, b) 25%igen und c) 50%igen emulgierbaren Konzentrates werden folgende Stoffe verwendet:

a) 10 Teile Wirkstoff,
3,4 Teile epoxydiertes Pflanzenöl,
3,4 Teile eines Kombinationsemulgators, bestehend aus Fettalkoholpolyglykoläther und Alkylarylsulfonat-Calcium-Salz,
40 Teile Dimethylformamid,
43,2 Teile Xylol;

b) 25 Teile Wirkstoff,
2,5 Teile epoxydiertes Pflanzenöl,
10 Teile eines Alkylarylsulfonat/Fettalkoholpolyglykoläther-Gemisches,
5 Teile Dimethylformamid,
57,5 Teile Xylol;

c) 50 Teile Wirkstoff,
4,2 Teile Tributylphenol-Polyglykoläther,
5,8 Teile Calcium-Dodecylbenzolsulfonat,
20 Teile Cyclohexanon,
20 Teile Xylol.

Aus solchen Konzentraten können durch Verdünnen mit Wasser Emulsionen jeder gewünschten Konzentration hergestellt werden.

Sprühmittel: Zur Herstellung eines a) 5%igen und b) 95%igen Sprühmittels werden die folgenden Bestandteile verwendet:

a) 5 Teile Wirkstoff,
1 Teil Epichlorhydrin,
94 Teile Benzin (Siedegrenzen 160-190°C);

b) 95 Teile Wirkstoff,
5 Teile Epichlorhydrin.

Beispiel 1: Herstellung von α-Isopropyl-4-chlorphenylessigsäure-(3-(4-chlorphenoxy)-α-cyano-benzyl)-ester

Zu einer Lösung von 6,8 g 3-(4-Chlorphenoxy-α-cyanobenzylalkohol in 20 ml Toluol werden unter Rühren bei 0-10°C nacheinander 3,15 ml Pyridin in 3 ml Toluol und 6,05 g α-Isopropyl-4-chlorphenylessigsäurechlorid in 5 ml Toluol zugetropft. Nach 10 stündigem Rühren bei Raumtemperatur wäscht man das Reaktionsgemisch mit Wasser 2n-Salzsäure und 3%iger Natriumbikarbonatlösung, trocknet die organische Schicht über Natriumsulfat und destilliert das Toluol ab. Man erhält die Verbindung der Formel

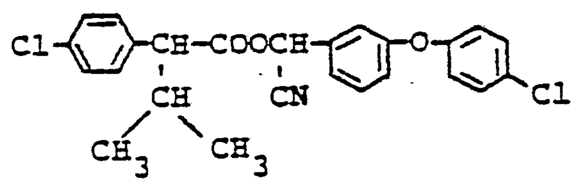

mit einem Brechungsindex von $n_D^{22°} = 1,5743$.

Auf analoge Weise werden auch folgende Verbindungen hergestellt:

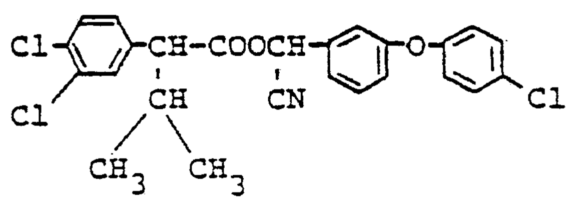

$n_D^{30}$ : 1,5789

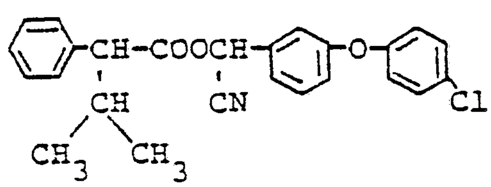

$n_D^{30}$ : 1,5671

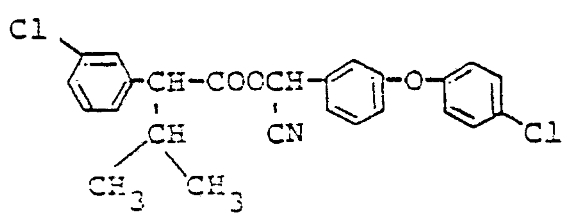

$n_D^{30}$ : 1,5714

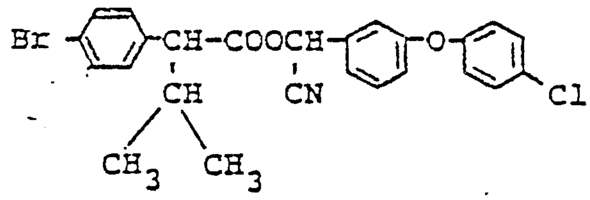

$n_D^{41}$: 1,5796

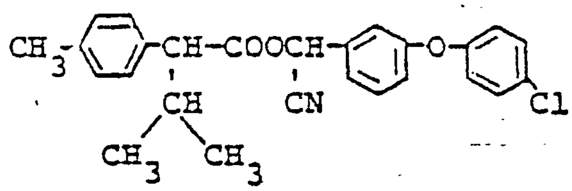

$n_D^{41}$: 1,5598

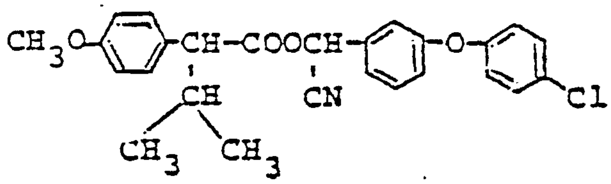

$n_D^{41}$: 1,5669

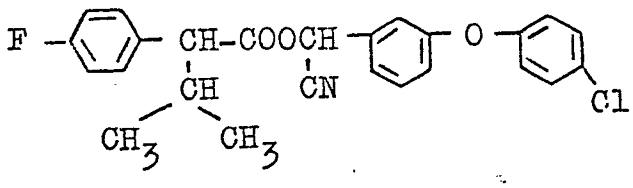

$n_D^{22}$: 1,5610

Beispiel 2: Insektizide Frassgift-Wirkung

Baumwollpflanzen wurden mit einer 0,05%igen wässrigen Wirkstoffemulsion (erhalten aus einem 10%igen emulgierbaren Konzentrat) besprüht.

Nach dem Antrocknen des Belages wurden die Baumwollpflanzen je mit Spodoptera littoralis- und Heliothis virescens-Larven $L_3$ besetzt. Der Versuch wurde bei 24°C und 60% relativer Luftfeuchtigkeit durchgeführt.

Verbindungen gemäss Beispiel 1 zeigten im obigen Test eine gute insektizide Frassgift-Wirkung gegen Spodoptera- und Heliothis-Larven.

Beispiel 3: Akarizide Wirkung

Phaseolus vulgaris Pflanzen wurden 12 Stunden vor dem Test auf akarizide Wirkung mit einem infestierten Blattstück aus einer Massenzucht von Tetranychus urticae belegt. Die übergelaufenen beweglichen Stadien wurden aus einem Chromatographiezerstäuber mit den emulgierten Testpräparaten derart besprüht, dass kein Ablaufen der Spritzbrühe eintrat. Nach zwei und 7 Tagen wurden Larven, Adulte und Eier unter dem Binokular auf lebende und tote Individuen ausgewertet und das Ergebnis in Prozenten ausgedrückt. Während der "Hältezeit" standen die behandelten Pflanzen in Gewächshauskabinen bei 25°C.

Verbindungen gemäss Beispiel 1 wirkten im obigen Test gegen Adulte, Larven und Eier von Tetranychus urticae.

Beispiel 4: Wirkung gegen Zecken

A) Rhipicephalus bursa.

Je 5 adulte Zecken bzw. 50 Zeckenlarven wurden in ein Glasröhrchen gezählt und für 1 bis 2 Minuten in 2 ml einer wässrigen Emulsion aus einer Verdünnungsreihe mit je 100, 10, 1 oder 0,1 ppm. Testsubstanz getaucht. Das Röhrchen wurde dann mit einem genormten Wattebausch verschlossen und auf den Kopf gestellt, damit die Wirkstoffemulsion von der Watte aufgenommen werden konnte.

Die Auswertung erfolgte bei den Adulten nach 2 Wochen und bei den Larven nach 2 Tagen. Für jeden Versuch liefen 2 Wiederholungen.

B) Boophilus microplus (Larven).

Mit einer analogen Verdünnungsreihe wie beim Test A) wurden mit je 20 sensiblen resp. OP-resistenten Larven Versuche durchgeführt. (Die Resistenz bezieht sich auf die Verträglichkeit von Diazinon).

Verbindungen gemäss Beispiel 1 wirkten in diesen Tests gegen Adulte und Larven von Rhipicephalus bursa und sensible resp. OP-resistente Larven von Boophilus microplus.

Beispiel 5: (Ausziehmethode)

Es wurde jeweils eine 0,4%ige Stammlösung einer der Verbindungen gemäss Beispiel 1 in Methylcellosolve bereitet. Dann wurde bei Zimmertemperatur eine wässrige Applikationsflotte hergestellt, die in 120 ml destilliertem Wasser 0,12 ml "Sandozin KB", 0,6 ml Ameisensäure 1:10 und 0,75 ml der jeweiligen 0,4%igen Stammlösung enthielt. Dann wurden 3 g Wollflanell-Gewebe mit heissem Wasser durchgenetzt und bei Zimmertemperatur eingegeben. Unter ständigem Umziehen des Wollmusters wurde die Badtemperatur innerhalb 20 Minuten auf 60°C erwärmt und 30 Minuten bei 60°C behandelt. Dann wurde abgekühlt, dass Wollmuster zweimal 3 Minuten mit destilliertem Wasser gespült, von Hand abgequetscht und an der Luft getrocknet. Die Wirkstoffkonzentration betrug 1000 ppm, berechnet auf das Wollgewicht.

Das so getrocknete Muster wurde der Mottenechtheitsprüfung (Frassschutz gegen Kleidermotte Tineola bisselliella Hum.), gemäss der Echtheitsprüfung gegen Larven des Pelzkäfers Attagenus piceus Ol. und Teppichkäfers (Anthrenus vorax Wat.), gemäss SNV 195902 unterworfen.

Es wurden jeweils Larven von Anthrenus vorax und 6 bis 7 Wochen alte Larven von Attagenus piceus zur Prüfung verwendet. Aus den behandelten Wollflanellmustern wurden Stücke gleicher Grösse ausgeschnitten und 14 Tage lang bei konstanter Temperatur (28°C) und konstanter relativer Luftfeuchtigkeit (65%) dem Angriff (Frass) von je 15 Larven des entsprechenden Schädlings ausgesetzt. Die Beurteilung erfolgte einerseits nach dem relativen Gewichtsverlust des Prüflings und andererseits nach der Anzahl noch lebender Organismen.

Die geprüften Verbindungen gemäss Beispiel 1 zeigten eine sehr gute Wirkung gegen die 3 verwendeten Schädlinge.

Beispiel 6: (Foulardmethode)

Es wurde jeweils eine 0,4%ige Stammlösung einer der Verbindungen gemäss Beispiel 1 in Methylcellosolve bereitet. 12,5 ml der jeweiligen Stammlösung wurden mit Methylcellosolve, welche 0,65G/l "Sandozin KB" enthält, auf 50 ml verdünnt (= Lösung Nr.1). 25 ml der Lösung Nr.1 werden mit Methylcellosolve, welche 0,5g/l "Sandozin KB" enthält, auf 50 ml verdünnt (= Lösung Nr.2). 25 ml von Lösung Nr.2 werden erneut mit Methylcellosolve, welche 0,5g/l "Sandozin KB" enthält, auf 50 ml verdünnt (= Lösung Nr.3).

Von den Lösungen Nr. 1, 2 und 3 wurden je 3 ml in Kristallisierschalen geleert und je eine geköderte Rondelle aus Wollflanell 3 Sekunden darin benetzt. Die feuchten Rondellen wurden anschliessend zwischen Aluminiumfolien fouladiert, und zwar derart, dass die abgequetschten Rondellen je 50% Flotte aufgenommen haben. Die Konzentrationen an Wirkstoff waren dann der Reihe nach 500 ppm, 250 ppm und 125 ppm für behandelte Rondellen aus den Lösungen Nr. 1, 2 und 3.

Die feuchten Rondellen wurden an der Luft getrocknet und den gleichen biologischen Prüfungen unterworfen, wie im Beispiel 5 beschrieben.

Die geprüften Verbindungen gemäss Beispiel 1 zeigten eine sehr gute Wirkung gegen alle 3 Schädlinge, auch bei der geringsten Konzentration von 125 ppm.

Patentansprüche

1. Ein α-Phenyl-α-cyclopropyl-acetat der Formel

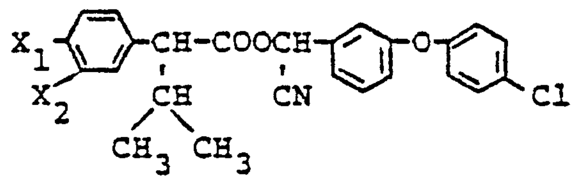

worin $X_1$ Wasserstoff, Halogen, $C_1$-$C_4$-Alkyl oder $C_1$-$C_4$-Alkoxy,
$X_2$ Wasserstoff oder Halogen oder
$X_1$ und $X_2$ zusammen Methylendioxy bedeuten.

2. Eine Verbindung gemäss Anspruch 1, worin $X_1$ und $X_2$ je Wasserstoff oder Chlor bedeuten.

3. Die Verbindung gemäss Anspruch 2 der Formel

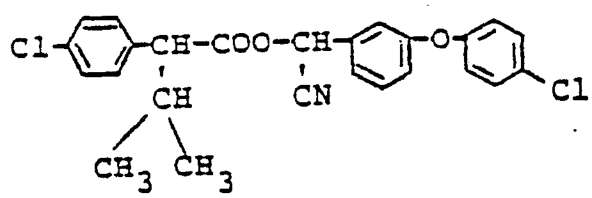

4. Die Verbindung gemäss Anspruch 2 der Formel

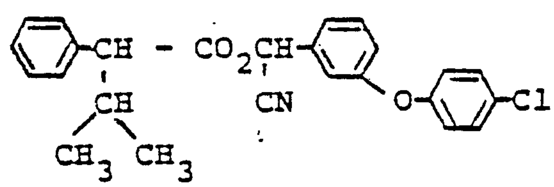

5. Die Verbindung gemäss Anspruch 2 der Formel

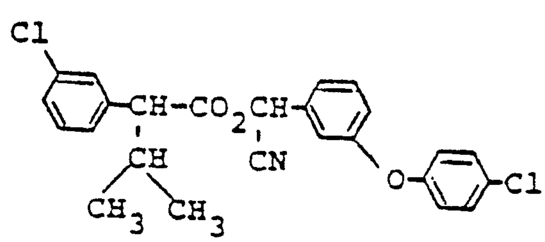

6. Die Verbindung gemäss Anspruch 2 der Formel

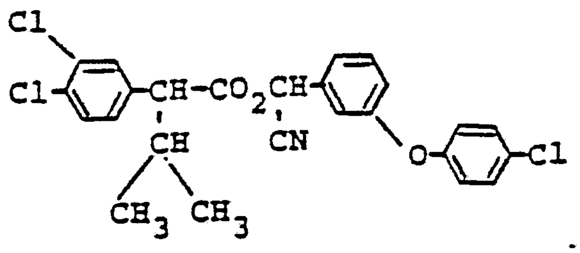

7. Verfahren zur Herstellung von Verbindungen gemäss Anspruch 1, dadurch gekennzeichnet, dass man eine Verbindung der Formel

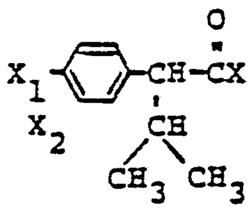

in Gegenwart eines säurebindenden Mittels mit der Verbindung der Formel

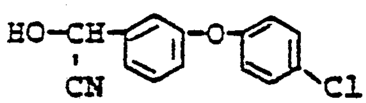

umsetzt, worin $X_1$ und $X_2$ die im Anspruch 1 angegebene Bedeutung haben und X für ein Halogenatom steht.

8. Ein Schädlingsbekämpfungsmittel, welches als aktive Komponente eine Verbindung gemäss Anspruch 1 und geeignete Träger- und/oder andere Zuschlagstoffe enthält.

9. Verwendung einer Verbindung gemäss Anspruch 1 zur Bekämpfung von verschiedenartigen tierischen und pflanzlichen Schädlingen.

10. Verwendung gemäss Anspruch 8 zur Bekämpfung von Insekten und Vertretern der Ordnung Akarina.

0009637

Europäisches Patentamt

# EUROPÄISCHER RECHERCHENBERICHT

Nummer der Anmeldung
EP 79103252.7

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | FR - A - 2 352 793 (CIBA-GEIGY AG.) * Patentansprüche * | 1, 7, 8, 9 |
| | DE - A - 2 651 341 (SHELL INTERNATIONALE RESEARCH MAATSCHAPPIJ B.V) * Patentansprüche; Seite 7, Zeilen 13 - 30* | 1, 7, 8, 9 |
| | DE - A - 2 737 297 (SUMITOMO CHEMICAL Co. Ltd.) * Patentansprüche; Seiten 5, 6 * | 1, 7, 8, 9 |
| | DE - A - 2 727 326 (IMPERIAL CHEMICAL INDUSTRIES Ltd.) * Patentansprüche 1, 7, 8, 9 * | 1, 7, 8, 9 |
| | DE - A - 2 615 435 (BAYER AG) * Seiten 1 - 3 * | 1, 7, 8, 9, 10 |
| | DE - A - 2 743 316 (AMERICAN CYANAMID Co.) * Seiten 1 - 4 * | 1, 7, 8, 9 |

KLASSIFIKATION DER ANMELDUNG (Int.Cl.$^{3}$)

C 07 C 121/75
C 07 D 317/60
A 01 N 37/34

RECHERCHIERTE SACHGEBIETE (Int. Cl.$^{3}$)

C 07 C 121/00
C 07 D 317/00
A 01 N 37/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsatze
E: kollidierende Anmeldung
D: in der Anmeldung angefuhrtes Dokument
L: aus andern Grunden angefuhrtes Dokument
&: Mitglied der gleichen Patentfamilie, ubereinstimmendes Dokument

X Der vorliegende Rechercnenbericht wurde fur alle Patentansprüche erstellt

| Recherchenort | Abschlußdatum der Recherche | Prüfer |
|---|---|---|
| WIEN | 29-11-1979 | HEIN |